# EUROPEAN PATENT APPLICATION

(11) **EP 0 763 359 A1**
(43) Date of publication of application: **19.03.1997**
(21) Application number: 96111154.9
(22) Date of filing: 11.07.1996
(51) Int. Cl.: A61K 31/495, A61K 31/19

(54) **Pharmaceutical composition for topical use**

(30) Priority: 14.07.1995 IT TO950607
(71) Applicant: R.R.A. S.r.l., 10025 Pino Torinese, Torino (IT)
(72) Inventor: Avataneo, Roberto, 10025 Pino Torinese, (Torino) (IT)
(74) Representative: Rambelli, Paolo

(57) **Abstract**

A pharmaceutical composition for topical use including, in association, as active ingredients:
a) a compound having a bactericidal activity chosen from the beta-lactam antibiotics and derivatives of 7-amino-1-cyclopropyl-4-oxo-1,4-dihydro-naphthyridin-3-carboxylic acid, their pharmaceutically acceptable salts and mixtures thereof; and
b) acetic acid.

## Description

The present invention concerns a pharmaceutical composition for topical use suitable for the treatment of dermatological and cutaneous diseases of bacterial origin and, in particular, for the treatment of otological diseases.

The subject of the invention is a pharmaceutical composition for topical use characterised in that it includes, in association, as active ingredients:
a) a compound having bactericidal activity chosen from the beta-lactam antibiotics, and derivatives of 7-amino-1-cyclopropyl-4-oxo-1,4-dihydro-naphthyridine-3-carboxylic acid, their pharmaceutically-acceptable salts and mixtures thereof; and
b) acetic acid.

The aforesaid 7-amino-1-cyclopropyl-4-oxo-1,4-dihydro-naphthyridine-3-carboxylic acid derivatives are known from patent No.EP-B-0 049 355 which describes a wide range of compounds having the general formula (I) (incorporated herein by reference), and which cites their broad-spectrum bactericidal activity which makes them useful in the treatment of systemic or localised bacterial infections.

In the field of the present invention, the use as an active anti-bacterial agent of a compound of the aforesaid class, belonging to the quinolone group of compounds, is particularly preferred, and the compound of 6-fluoro-7-piperazine-1-cyclopropyl-4-oxo-1,4-dihydro-quinoline-3-carboxyliacid or its pharmaceutically-acceptable salts, known by the common international name of CIPROFLOXACIN is more particularly preferred.

The use of piperacillin, (Merck Index X Ed. 7335), particularly sodium piperacillin, as the beta-lactam antibiotic is preferred.

It has been established that the combination of the active antibacterial agent, chosen from the aforesaid compounds and, in particular, from CIPROFLOXACIN or sodium PIPERACILLIN, and acetic acid in a preparation for topical use, gives rise to a drug which is unexpectedly effective in treating dermatological diseases of a bacterial nature. Particularly unexpected is the effectiveness of the drug obtained in this way in treating acute and/or chronic bacterial diseases of the outer ear (such as phlegmon of the auditory canal, and furunculosis), and the middle ear, even in the presence of perforation of the tympanic membrane.

The result is particularly surprising as known antibiotic drugs, such as neomycin and gentamicin, which are active against Pseudomonas aeruginosa, are inactive in the specific treatment to which the invention refers.

In the formulation that is the subject of the invention, the active antibacterial agent is generally present in a concentration of 1% to 8% by weight of the weight of the composition, and the acetic acid is preferably present in a concentration of 1% to 5% by weight, more preferably approximately 2% to 4% by weight; the pH of the composition is preferably between 2.5 and 5, more preferably between 3 and 4.5.

Known pharmaceutically-acceptable vehicles may be used as the vehicle for the preparation for topical use. In particular, the use of an aqueous, or possibly alcoholic, vehicle in solution form is preferred.

It is understood that the pharmaceutical composition may include the usual additives and excipients, commonly used in formulations for topical use such as, for example, preservatives, stabilisers, surfactants and aromatisers. Formulations including the active agents a) and b) in combination with an amino acid such as glycine (glycocholate) are particularly advantageous.

### Examples

Solutions in an aqueous vehicle for topical use were prepared including:
Formulation A)
   CIPROFLOXACIN: 2% by weight
   acetic acid (official pharmacopoeia): 2% by weight
   The solution is made up to 100% with the aqueous vehicle.
Formulation B)
   SODIUM PIPERACILLIN: 4% by weight
   acetic acid (official pharmacopoeia): 2% by weight

### Case study

A case study of patients subjected to treatment with the aforesaid formulations is reported below:
I. Patient with phlegmon of the right auditory canal and thickening of the tympanic membrane which also presented a small anterior perforation; positive otological test for Pseudomonas aeruginosa; subjected to surgical intervention to clean the auditory canal (also presented osteitis of the auditory canal and thinning of the tympanic membrane).
   Medical treatment with drug A was conducted for two weeks post-surgery with the administration of seven drops, twice daily, leading to full recovery with healing of the tympanic membrane.
II. Patient with a recurring polypoidal postero-superior portion of the left auditory canal following left enlargement tympanoplasty with a positive test for Pseudomonas aeruginosa; treated by removing neoformation polypoids under local anaesthetic and medical treatment for two weeks with drug A at a dose of ten drops, twice daily, with full recovery.
III. Patient with acute right otitis media with perforation of the tympanic membrane; positive test for Pseudomonas aeruginosa; subjected to local treatment with drug A for two weeks with the administration of ten drops of the drug, twice daily, leading to full recovery with healing of the tympanic membrane.
IV. Patient with left serous otitis media with a rear perforation of the tympanic membrane; subjected to medical treatment with drug A for ten days with the administration of ten drops of the drug, twice daily, leading to full recovery with healing of the tympanic membrane.
V. 12 year-old patient with post-surgical infection after tympanocentesis with aspiration; subjected to local treatment with drug A for two weeks with the administration of four drops, twice daily, leading to full recovery with healing of the tympanic membrane.
VI. Patient with long-standing right phlegmonic otitis externa unresponsive to any parenterally-administered treatment; positive test for Pseudomonas aeruginosa; subjected to local treatment with drug A for two weeks with the administration of twelve drops, twice daily, leading to full recovery.
VII. Drug A was also used in the post-surgical treatment, after tympanoplasty, without any parenterally administered treatment; re-infection was not seen in any case.
VIII. 33 year-old male patient.
   Diagnosis: recurrent phlegmonic otitis
   Treatment: three cycles of treatment lasting for ten days
   with the administration of drug B leading to recovery.

   When the disease reappeared after two months, the patient was subjected to two new treatment cycles with full recovery.
IX. 66 year-old female patient.
   Diagnosis: chronic perforated right otitis media in the
   acute secreting phase
   Treatment: drug B for ten days leading to full recovery.
X. 51 year-old male patient.
   Diagnosis: phlegmon of the right auditory canal with osteitis and paracentral perforation of the right tympanic membrane.
   Surgical intervention in the form of courettage of the right auditory canal and thinning of the right tympanic membrane.
   The patient was subjected during the post-surgery phase to two cycles of treatment with drug A for twenty days for the presence of serous otorrhoea. The otorrhoea recurred one month after the second cycle with a positive test for Alcaligens Xyloroxidois. Local treatment with drug B for twenty days was followed by a negative test result and recovery.
XI. 56 year-old male patient.
   Diagnosis: mucopurulent left perforated acute otitis media; unresponsive to two cycles of orally-administered azitromycin.
   Treatment: drug B for fifteen days leading to recovery.
XII. 23 year-old female patient.
   Diagnosis: left phlegmonic otitis; right otitis interna with initial perforation of the tympanic membrane.
   The patient was unresponsive to treatment with topically-applied Ceftriaxone in acetic acid.
   Treatment: drug B for twelve days leading to full recovery.
XIII. 25 year-old female patient.
   Diagnosis: right otitis media, acute serocatarrh with laceration of the tympanic membrane.
   Treatment: drug B for ten days leading to recovery.
XIV. 51 year-old male patient.
   Diagnosis: acute right otitis externa with perforation of the tympanic membrane; positive test for Pseudomonas aeruginosa.
   Treatment: drug B; two cycles of ten days with an interval of ten days leading to complete recovery.
XV. 29 year-old female patient.
   Diagnosis: perforated right acute otitis media; positive test for beta-hæmolytic streptococcus + Escherichia-coli; unresponsive to treatment with amoxycillin per os.
   Treatment: drug B for ten days; two treatment cycles with an interval of ten days between the first and second cycles leading to full recovery.
XVI. 61 year-old male patient.
   Diagnosis: left phlegmonic otitis with perforation of the tympanic membrane.
   Treatment with the quinolo compound per os and topical acetic acid for ten days with no change.
   Treatment: drug B for ten days; two treatment cycles with an interval of ten days and recovery.

None of the aforesaid cases were supported by any parenteral treatment.

The invention therefore makes available to the physician a pharmaceutical preparation which allows the treatment of diseases of the ear which have caused the perforation or destruction of the tympanic membrane, and which could not be treated by the topical application of the currently available drugs.

The currently available otological solutions (drops) for topical use have, at best, an analgesic, and hence purely a symptomatic, effect. The rare cortico-antibiotic preparations that are commercially available may be administered only in respect of diseases of the outer ear where tnere is no perforation of the tympanic membrane.

In addition, the pharmaceutical composition according to the invention may be administered to patients of any age, even children, since the topical administration eliminates all of the damage and collateral effects deriving from systemic administration; topical administration eliminates the hepatic and renal contra-indications, and there is no harmful effect on the digestive tract.

## Claims

1. A pharmaceutical composition for topical use including, in association, as active ingredients:
a) a compound having a bactericidal activity chosen from the beta-lactam antibiotics and derivatives of 7-amino-1-cyclopropyl-4-oxo-1,4-dihydro-naphthyridin-3-carboxylic acid, their pharmaceutically-acceptable salts and mixtures thereof; and
b) acetic acid.

2. A pharmaceutical composition according to claim 1, in which the compound in the class cited in claim 1 having a bactericidal activity, belongs to the quinolone group.

3. A pharmaceutical composition according to claim 2, in which the said compound having a bactericidal activity is CIPROFLOXACIN.

4. A pharmaceutical composition according to claim 1, in which the compound having a bactericidal activity is piperacillin.

5. A pharmaceutical composition according to any of claims 1 to 4, in which the bactericide compound is present in a concentration of 1% to 8% by weight, preferably from 1% to 6% by weight.

6. A pharmaceutical composition according to any of claims 1 to 5, including a pharmaceutically-acceptable vehicle for topical use.

7. A pharmaceutical composition according to claim 6, in which the said vehicle is an aqueous vehicle.

8. A pharmaceutical composition according to any of claims 1 to 7, in which the acetic acid is present in a concentration of from 1% to 5% by weight of the weight of the formulation.

9. A pharmaceutical composition according to any preceding claim for the treatment of dermatological diseases of a bacterial nature.

10. A pharmaceutical composition according to any of claims 1 to 8 for the treatment of otological diseases.

11. A pharmaceutical composition according to claim 10 for the treatment of otological diseases where there is perforation of the tympanic membrane.

12. The use of a compound having a bactericidal activity chosen from the beta-lactam antibiotics and derivatives of 7-amino-1-cyclopropyl-4-oxo-1,4-dihydro-naphthyridin-3-carboxylic acid, their pharmaceutically-acceptable salts and mixtures thereof, in association with acetic acid for the preparation of a medication for topical use for the treatment of dermatological and otological diseases of a bacterial nature.

13. Use according to claim 12, in which the compound having a bactericide action is CIPROFLOXACIN.

14. Use according to claim 12, in which the compound is piperacillin.
